(19) Europäisches Patentamt
European Patent Office
Office européen des brevets

(11) Veröffentlichungsnummer: **0 225 522**
**A1**

(12) # EUROPÄISCHE PATENTANMELDUNG

(21) Anmeldenummer: **86116123.0**

(22) Anmeldetag: **21.11.86**

(51) Int. Cl.⁴: **C 07 D 487/04**, A 61 K 31/50
// (C07D487/04, 237:00, 235:00)

(30) Priorität: **03.12.85 DE 3542661**

(43) Veröffentlichungstag der Anmeldung: **16.06.87 Patentblatt 87/25**

(84) Benannte Vertragsstaaten: **AT BE CH DE ES FR GB GR IT LI NL SE**

(71) Anmelder: **BAYER AG, Konzernverwaltung RP Patentabteilung, D-5090 Leverkusen 1 Bayerwerk (DE)**

(72) Erfinder: **Meyer, Horst, Dr., Henselweg 11, D-5600 Wuppertal 1 (DE)**
Erfinder: **Ingendoh, Axel, Dr., Unterste Dillenberg 18, D-5620 Velbert 15 (DE)**
Erfinder: **Garthoff, Bernward, Dr., Händelstrasse 22, D-4010 Hilden (DE)**
Erfinder: **Hirth, Claudia, Dr., Stockmannsmühle 127, D-5600 Wuppertal 1 (DE)**

(54) **Imidazopyridazinalkensäureamide, Verfahren ihrer Herstellung, Zwischenprodukte zu ihrer Herstellung.**

(57) Die Erfindung betrifft Imidazopyridazinalkensäureamide, der allgemeinen Formel (I)

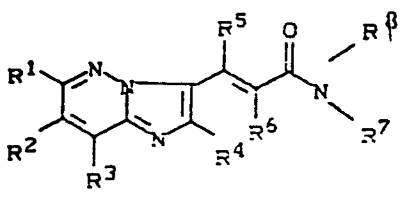

(I)

mit den in der Beschreibung angegebenen Bedeutungen, Verfahren zu ihrer Herstellung, Zwischenprodukte zu ihrer Herstellung sowie ihre Verwendung zur Behandlung von Krankheiten, insbesondere als Antihypertensiva, Diuretika und Saluretika.

EP 0 225 522 A1

0225522

BAYER AKTIENGESELLSCHAFT 5090 Leverkusen, Bayerwerk
Konzernverwaltung RP
Patentabteilung Si/ABc

# Imidazopyridazinalkensäureamide

Die Erfindung betrifft Imidazopyridazinalkensäureamide, Verfahren zu ihrer Herstellung, Zwischenprodukte zu ihrer Herstellung sowie ihre Verwendung zur Behandlung von Krankheiten, insbesondere als Antihypertensiva, Diuretika und Saluretika.

Aus US-PS 4 444 770 ist bekannt, daß Imidazoazolalkensäureamide antihypertensive und diuretische Wirkung besitzen.

Die vorliegende Erfindung betrifft neue Imidazopyridazinalkensäureamide der allgemeinen Formel (I)

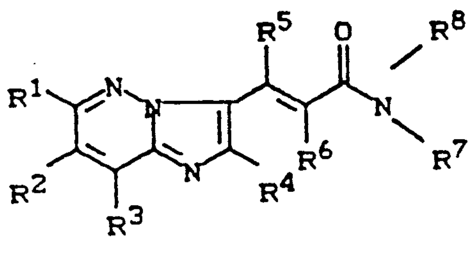

(I)

in welcher

$R^1$ - für Wasserstoff oder
- für Alkoxy oder
- für gegebenenfalls durch Phenyl, Cyano, Hydroxy oder Halogen substituiertes Alkyl oder Alkenyl steht, wobei die Kohlenstoffkette des Alkylrestes gegebenenfalls durch O, S, NH, N-Alkyl, N-Aryl oder N-Aralkyl unterbrochen ist, oder

- für einen Aryl- oder Heterocyclylrest steht, der gegebenenfalls ein, zwei oder drei gleiche oder verschiedene Substituenten aus der Gruppe Alkyl, Alkoxy, Alkylthio, Trifluormethyl, Trifluormethoxy, Halogen, Nitro, Cyano oder Alkylsulfonyl trägt, oder

- für eine Gruppe $SO_n$-Alkyl steht, worin n 0, 1 oder 2 bedeutet, oder
- für eine Gruppe der Formel

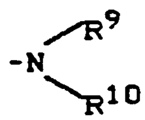 oder $-\underset{\underset{O}{\|}}{C}-R^{11}$ steht,

wobei
$R^9$, $R^{10}$ - gleich oder verschieden sind und
- für Wasserstoff, oder
- für gegebenenfalls durch O, S, NH oder N-Alkyl in der Kette unterbrochenes Alkyl stehen, oder
- für Aryl oder Aralkyl stehen, oder
- für Amino, Alkylamino oder Arylamino stehen,

oder

$R^9$ und $R^{10}$ gemeinsam mit dem Stickstoffatom einen 5- bis 7-gliedrigen, gesättigten oder ungesättigten Ring bilden, der gegebenenfalls ein oder zwei Sauerstoff-, Schwefel- und/oder Stickstoffatome als weitere Heteroatome enthalten kann und wobei diese Stickstoffatome gegebenenfalls durch Alkyl, Aryl, Aralkyl, Hdroxyalkyl, Acyl, Alkylaminocarbonyl oder Arylaminocarbonyl substituiert sind,

und wobei

$R^{11}$
- für Wasserstoff,
- für Hydroxy,
- für Alkyl oder Alkenyl,
- für Alkoxy oder Alkenyloxy,
- für Aryloxy oder Aralkoxy steht, oder
- für eine Gruppe der Formel

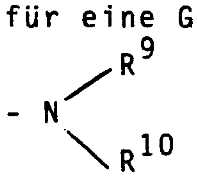

steht,

wobei
$R^9$ und $R^{10}$ die bereits angegebene Bedeutung haben,

$R^2$, $R^3$
- gleich oder verschieden sind und
- für Wasserstoff oder Alkyl stehen,

$R^4$
- für einen Aryl- oder Heterocyclylrest steht, der gegebenenfalls bis zu zweifach gleich oder verschieden durch Alkyl, Halogen, Nitro, Cyano, Alkoxy, Alkylsulfonyl oder Trifluormethyl substituiert ist,

$R^5$ - für Wasserstoff, Alkyl oder Trifluormethyl steht,

$R^6$ - für Wasserstoff, Cyano, Halogen oder
- für Alkoxy, Alkyl oder Sulfonylalkyl, oder
- für die Gruppe $-\underset{\underset{O}{\|}}{C}-R^{11}$ steht, wobei

$R^{11}$ die bereits angegebene Bedeutung hat,

und

$R^7$, $R^8$ - gleich oder verschieden sind und
- für Wasserstoff,
- für Aryl, oder
- für einen geradkettigen, verzweigten oder cyclischen gesättigten oder ungesättigten Kohlenwasserstoffrest mit bis zu 10 C-Atomen stehen, der gegebenenfalls durch O, S, NH, N-Alkyl, N-Aryl oder N-Aralkyl unterbrochen ist, und der gegebenenfalls substituiert ist durch Hydroxy, Alkoxy, Trifluormethyl, Halogen, Phenyl, Alkoxycarbonyl oder Dialkylamino, wobei die beiden Alkylreste gegebenenfalls gemeinsam mit dem Stickstoffatom einen 5 - 7-gliedrigen Ring bilden, der gegebenenfalls durch O, S, NH oder N-Alkyl unterbrochen ist und wobei die vorgenannten Alkyl- sowie Phenylreste ihrerseits gegebenenfalls durch Halogen, Trifluormethyl, Alkyl, Aryl, Aralkyl, Alkoxy, Alkylthio, Alkylsulfonyl oder Cyano substituiert sind,

oder

$R^7$ und $R^8$ gemeinsam mit dem Stickstoffatom einen 3- bis 8-gliedrigen, gesättigten oder ungesättigten Ring bilden, der gegebenenfalls ein oder zwei O, S, N, NH, N-Alkyl, N-Aryl oder N-Aralkyl enthält, der gegebenenfalls bis zu drei Substituenten aus der Reihe Alkyl, Halogen, Aryl, Aralkyl, Alkoxycarbonyl, Hydroxyalkyl, Alkoxyalkyl oder Trifluormethyl tragen kann, oder der mit einem gegebenenfalls substituierten aromatischen Ring kondensiert sein kann,

sowie ihre physiologisch unbedenklichen Salze, sowie ihre stereoisomeren Formen in Form von Enantiomeren, Diastereomeren und E/Z-Isomeren.

Die erfindungsgemäßen Imidazopyridazinalkensäureamide haben gute diuretische sowie saluretische Wirkung.

Im Rahmen der obigen Substituentendefinition steht Alkyl im allgemeinen für einen geradkettigen, verzweigten oder cyclischen gesättigten Kohlenwasserstoff mit bis zu 10 C-Atomen, bevorzugt mit bis zu 8 C-Atomen.

Alkenyl steht im allgemeinen für einen geradkettigen, verzweigten oder cyclischen Kohlenwasserstoffrest mit bis zu 10 C-Atomen und bis zu zwei Doppelbindungen, bevorzugt mit bis zu 8 C-Atomen und einer Doppelbindung.

Alkoxy steht im allgemeinen für einen geradkettigen oder verzweigten Alkylrest mit bis zu 10 C-Atomen, bevorzugt mit bis zu 8 C-Atomen, der über ein Sauerstoffatom gebunden ist.

Aryl steht im allgemeinen für einen aromatischen Kohlenwasserstoffrest mit bis zu 14 C-Atomen, bevorzugt für Phenyl oder Naphthyl.

Aralkyl steht im allgemeinen für einen Phenylrest, der über eine Alkylkette mit bis zu 6 C-Atomen gebunden ist. Bevorzugt steht Aralkyl für Benzyl oder Phenethyl.

Heterocyclyl steht im allgemeinen für einen 5- bis 7-gliedrigen Ring, der als Heteroatome bis zu 3 Sauerstoff-, Schwefel- und/oder Stickstoffatome enthalten kann, der gesättigt oder ungesättigt sein kann und der gegebenenfalls durch Halogen, Hydroxy, Amino, Alkylamino, Dialkylamino, Alkyl, Trifluormethyl oder Alkoxy substituiert sein kann. Bevorzugt steht Heterocyclyl für Reste wie Pyridyl, Thienyl, Furyl, Pyrrolyl, Pyrimidyl, Pyrazinyl, Pyridazinyl, Chinolyl, Isochinolyl, Piperidinyl, Piperazinyl, Morpholinyl oder Thiomorpholinyl.

Halogen steht im allgemeinen für Fluor, Chlor, Brom oder Iod, bevorzugt für Fluor, Chlor oder Brom.

Bevorzugte Imidazopyridazinalkensäureamide der Formel I sind solche, in denen

$R^1$ - für Wasserstoff oder

- für $C_1$-$C_6$-Alkoxy steht, oder
- für gegebenenfalls durch Phenyl, Cyano, Hydroxy, Fluor, Chlor oder Brom substituiertes $C_1$-$C_6$-Alkyl, $C_2$-$C_6$-Alkenyl, Cyclopentyl oder Cyclohexyl steht, wobei die Kohlenstoffkette des Alkylrestes gegebenenfalls durch O, S, NH, N-$C_1$-$C_6$-Alkyl, N-Phenyl oder N-Benzyl unterbrochen ist, oder

- für einen Phenyl-, Naphthyl-, Furyl-, Thienyl-, Pyrimidyl-, Pyrazinyl-, Pyridyl-, Chinolyl- oder Isochinolylrest steht, der gegebenenfalls durch ein oder zwei gleiche oder verschiedene Substituenten der Reihe $C_1$-$C_6$-Alkyl, $C_1$-$C_6$-Alkoxy, Fluor, Chlor, Brom, Nitro, Cyano, Trifluormethyl, Trifluormethoxy oder $C_1$-$C_6$-Alkylsulfonyl substituiert ist, oder

- für eine Gruppe $SO_n$-Alkyl steht,

wobei
n für 0 oder 2 steht und Alkyl bis zu 6 C-Atome hat,

oder
- für einen Gruppe der Formel 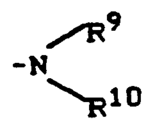 oder

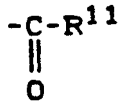 steht,

wobei

$R^9$, $R^{10}$ - gleich oder verschieden sind und
- für Wasserstoff oder
- für $C_1$-$C_6$-Alkyl stehen, das gegebenenfalls durch O, S, NH oder N-$C_1$-$C_6$-Alkyl unterbrochen ist, oder

- für Phenyl, Naphthyl oder Benzyl stehen, oder
- für Amino, $C_1$-$C_4$-Alkylamino oder Phenylamino stehen,

oder

$R^9$ und $R^{10}$ gemeinsam mit dem Stickstoffatom einen 5- bis 7-gliedrigen, gesättigten oder ungesättigten Ring bilden, der gegebenenfalls ein oder zwei Sauerstoff-, Schwefel- oder Stickstoffatome als weitere Heteroatome enthalten kann, und wobei diese Stickstoffatome gegebenenfalls durch $C_1$-$C_6$-Alkyl, $C_1$-$C_6$-Hydroxyalkyl, Phenyl, Benzyl, Benzoyl, Acetyl, $C_1$-$C_4$-Alkylaminocarbonyl oder Phenylaminocarbonyl substituiert sind,

$R^{11}$
- für Wasserstoff,
- für Hydroxy,
- für $C_1$-$C_6$-Alkyl, $C_2$-$C_6$-Alkenyl,
- für $C_1$-$C_6$-Alkoxy, $C_2$-$C_6$-Alkenyloxy,
- für Phenoxy oder Benzyloxy steht, oder
- für eine Gruppe der Formel

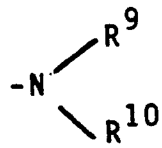

steht,

wobei
$R^9$ und $R^{10}$ die oben angegebene Bedeutung haben,

$R^2$, $R^3$
- gleich oder verschieden sind und
- für Wasserstoff oder $C_1$-$C_6$-Alkyl stehen,

$R^4$
- für gegebenenfalls durch $C_1$-$C_6$-Alkyl, Fluor, Chlor, Brom, Nitro, Cyano, $C_1$-$C_6$-Alkoxy, $C_1$-$C_6$-Alkylsulfonyl oder Trifluoromethyl substi-

tuiertes Phenyl, Naphthyl, Thienyl, Furyl, Pyridyl, Pyrimidyl, Chinolyl oder Isochinolyl steht,

$R^5$ - für Wasserstoff, Trifluormethyl oder $C_1$-$C_6$-Alkyl steht,

$R^6$ - für Wasserstoff, Cyano, Fluor, Chlor, Brom,
- für $C_1$-$C_6$-Alkyl oder
- für die Gruppe $-\underset{\underset{O}{\|}}{C}-R^{11}$ steht, wobei $R^{11}$ die bereits angegebene Bedeutung hat,

und

$R^7$, $R^8$ - gleich oder verschieden sind und
- für Wasserstoff,
- für Phenyl oder
- für einen geradkettigen, verzweigten oder cyclischen Kohlenwasserstoffrest mit bis zu 8 C-Atomen stehen, der gegebenenfalls durch O, S, NH, N-$C_1$-$C_6$-Alkyl, N-Phenyl oder N-Benzyl unterbrochen ist, und der gegebenenfalls substituiert ist durch Hydroxy, $C_1$-$C_6$-Alkoxy, Trifluormethyl, Fluor, Chlor, Brom, Phenyl, $C_1$-$C_6$-Alkoxycarbonyl, Di-$C_1$-$C_6$-Alkylamino, Piperidin, Piperazin, Morpholin, Thiomorpholin, N-Methylpiperazin oder Pyrrolidin, wobei die vorgenannten Alkylsowie Phenylreste ihrerseits gegebenenfalls durch Fluor, Chlor, Brom, Trifluormethyl, $C_1$-$C_6$-

Alkyl, Phenyl, Benzyl, $C_1$-$C_6$-Alkoxy, $C_1$-$C_6$-Alkylsulfonyl oder Cyano substituiert sind,

oder

$R^7$ und $R^8$ gemeinsam mit dem Stickstoffatom einen 5- bis 7-gliedrigen Ring bilden, der gegebenenfalls 1 oder 2 weitere Heteroatome aus der Gruppe Sauerstoff, Schwefel oder Stickstoff enthält, wobei der Stickstoff gegebenenfalls durch Wasserstoff, $C_1$-$C_6$-Alkyl, Phenyl oder Benzyl substituiert ist und wobei der Ring durch bis zu drei gleiche oder verschiedene Substituenten aus der Reihe $C_1$-$C_6$-Alkyl, Fluor, Chlor, Brom, Phenyl, Benzyl, $C_1$-$C_6$-Alkoxycarbonyl, Hydroxy-$C_1$-$C_6$-alkyl, $C_1$-$C_4$-Alkoxy-$C_1$-$C_6$-alkyl oder Trifluormethyl substituiert sein kann, oder wobei der Ring mit einem gegebenenfalls substituierten aromatischen Ring kondensiert sein kann,

sowie ihre physiologisch unbedenklichen Salze, sowie ihre stereoisomeren Formen in Form von Enantiomeren, Diastereomeren und E/Z-Isomeren.

Besonders bevorzugt sind Verbindungen der allgemeinen Formel I, in welcher

$R^1$ - für Wasserstoff,
- für geradkettiges oder verzweigtes $C_1$-$C_4$-Alkoxy oder

- für gegebenenfalls durch Fluor, Chlor oder Hydroxy substituiertes, geradkettiges oder verzweigtes $C_1$-$C_6$-Alkyl steht, wobei die Alkylkette gegebenenfalls durch O, S, NH, N-$C_1$-$C_4$-Alkyl, N-Phenyl oder N-Benzyl unterbrochen ist, oder

- für einen Phenyl-, Naphthyl-, Furyl-, Thienyl, Pyrimidyl- oder Pyridylrest steht, oder

- für eine Gruppe der Formel 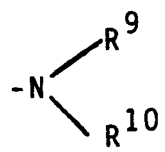 oder 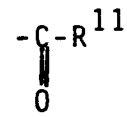

steht,
wobei

$R^9$, $R^{10}$ - gleich oder verschieden sind und
- für Wasserstoff oder
- für geradkettiges oder verzweigtes $C_1$-$C_6$-Alkyl stehen, das gegebenenfalls durch O, S, NH oder N-$C_1$-$C_3$-Alkyl unterbrochen ist, oder
- für Phenyl oder Benzyl stehen, oder
- für Amino stehen,

oder

$R^9$ und $R^{10}$ gemeinsam mit dem Stickstoffatom einen 5- bis 6-gliedrigen, gesättigten oder ungesättigten Ring bilden, der ein Sauerstoff-, Schwefel- und/oder bis zu zwei Stickstoffatome als weitere Heteroatome enthalten kann, und wobei die Stickstoffatome gegebenenfalls durch $C_1$-$C_3$-Alkyl, $C_1$-$C_3$-Hydroxyalkyl, Phenyl, Benzyl, Benzoyl, Acetyl, $C_1$-$C_3$-Alkylaminocarbonyl oder Phenylaminocarbonyl substituiert sind,

und wobei

$R^{11}$ - für Wasserstoff,
- für Hydroxy,
- für geradkettiges oder verzweigtes $C_1$-$C_4$-Alkyl,
- für geradkettiges oder verzweigtes $C_1$-$C_4$-Alkoxy oder
- für Phenoxy oder Benzyloxy steht,

$R^2$, $R^3$ - gleich oder verschieden sind und
- für Wasserstoff oder
- für geradkettiges oder verzweigtes $C_1$-$C_4$-Alkyl stehen,

$R^4$ - für gegebenenfalls durch Fluor, Chlor, Nitro, Cyano, $C_1$-$C_4$-Alkyl, $C_1$-$C_4$-Alkoxy oder Trifluormethyl substituiertes Phenyl, Thienyl oder Pyridyl steht,

$R^5$, $R^6$ - gleich oder verschieden sind und
- für Wasserstoff oder
- für geradkettiges oder verzweigtes $C_1$-$C_4$-Alkyl stehen,

und

$R^7$, $R^8$ - gleich oder verschieden sind und
- für Wasserstoff,
- für Phenyl oder

- für einen geradkettigen, verzweigten oder cyclischen Kohlenwasserstoffrest mit bis zu 6 C-Atomen stehen, der gegebenenfalls durch O, S, NH, N-$C_1$-$C_4$-Alkyl, N-Phenyl oder N-Benzyl unterbrochen ist, und der gegebenenfalls substituiert ist durch Hydroxy, $C_1$-$C_4$-Alkoxy, Trifluormethyl, Fluor, Chlor, Phenyl, Di-$C_1$-$C_4$-alkylamino, Piperidin, Piperazin, N-Methylpiperazin, Morpholin oder Pyrrolidin, wobei die vorgenannten Alkyl- oder Phenylreste ihrerseits gegebenenfalls durch Fluor, Chlor, Trifluormethyl, $C_1$-$C_4$-Alkyl oder $C_1$-$C_4$-Alkoxy substituiert sind,

oder

$R^7$ und $R^8$ gemeinsam mit dem Stickstoffatom einen 5- bis 7-gliedrigen Ring bilden, der gegebenenfalls ein oder zwei weitere Heteroatome aus der Gruppe Sauerstoff, Schwefel oder Stickstoff enthält, wobei dieser Stickstoff gegebenenfalls durch Wasserstoff, $C_1$-$C_4$-Alkyl, Phenyl oder Benzyl substituiert ist und wobei der Ring durch bis zu zwei gleiche oder verschiedene Substituenten aus der Reihe $C_1$-$C_4$-Alkyl, Fluor, Chlor, $C_1$-$C_4$-Hydroxyalkyl, $C_1$-$C_4$-Alkoxy-$C_1$-$C_4$-alkyl oder Trifluormethyl substituiert sein kann oder wobei der Ring mit einem aromatischen Ring kondensiert sein kann,

sowie ihre physiologisch unbedenklichen Salze, sowie ihre stereoisomeren Formen in Form von Enantiomeren, Diastereomeren und E/Z-Isomeren.

Je nach Wahl der Ausgangssubstanzen können die erfindungsgemäßen Verbindungen in stereoisomeren Formen existieren, die sich entweder wie Bild und Spiegelbild (Enantiomere) oder die sich nicht wie Bild und Spiegelbild (Diastereomere) verhalten. Sowohl die Antipoden als auch die Racemformen als auch die Diastereomerengemische sind Gegenstand der vorliegenden Erfindung. Die Racemformen lassen sich ebenso wie die Diastereomeren in bekannter Weise in die stereoisomer einheitlichen Bestandteile trennen (vgl. z.B. E.L. Eliel, Stereochemistry of Carbon Compounds, McGraw Hill (1962)).

Darüber hinaus werden die durch die Doppelbindung der Seitenkette möglichen E/Z-Isomeren beansprucht, die nach bekannten Verfahren hergestellt oder nach bekannten Verfahren ineinander überführt werden können.

Die erfindungsgemäßen Verbindungen können in Form ihrer Salze vorliegen. Im allgemeinen sind dies Salze der erfindungsgemäßen Stoffe mit anorganischen oder organischen Säuren. Bevorzugt sind jedoch die physiologisch unbedenklichen Salze der erfindungsgemäßen Stoffe mit anorganischen und organischen Säuren. Als Beispiele seien genannt: Hydrohalogenide, Hydrogensulfate, Sulfate, Hydrogenphosphate, Acetate, Maleate, Citrate, Fumarate, Tartrate, Lactate oder Benzoate.

Die erfindungsgemäßen Verbindungen der allgemeinen Formel (I) erhält man, wenn man

[A] Carbonylverbindungen der allgemeinen Formel (II),

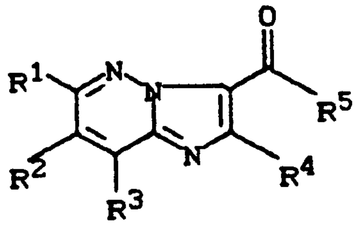

(II)

in welcher

$R^1$ - $R^5$ die oben angegebene Bedeutung haben,

mit Phosphonaten der allgemeinen Formel (III)

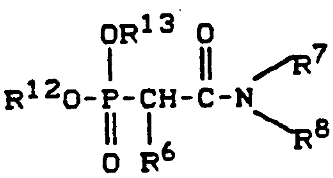

(III)

in welcher

$R^6$ - $R^8$ die oben angegebene Bedeutung haben und

$R^{12}$, $R^{13}$ für gegebenenfalls substituiertes Alkyl oder Aralkyl stehen,

in Gegenwart starker Basen in inerten organischen Lösungsmitteln bei Temperaturen zwischen +20° C und +200° C, bevorzugt zwischen +20° C und +150° C umsetzt,

oder wenn man

[B] Carbonylverbindungen der allgemeinen Formel (II) mit Acetamiden der allgemeinen Formel (IV),

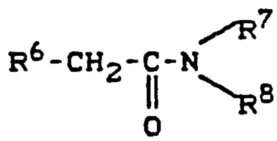

(IV)

in welcher

$R^6$ - $R^8$ die oben angegebene Bedeutung haben,

in Gegenwart von sauren oder basischen Katalysatoren und gegebenenfalls in Anwesenheit inerter organischer Lösungsmittel bei Temperaturen zwischen + 20 °C und + 200 °C, bevorzugt zwischen + 20 °C und + 150 °C umsetzt,

oder wenn man

[C] Alkencarbonsäuren der allgemeinen Formel (V)

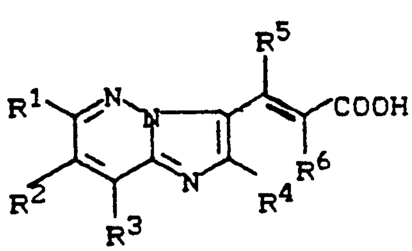

(V)

in welcher

$R^1$ -$R^6$ die oben angegebene Bedeutung haben,

mit Aminen der allgemeinen Formel (VI)

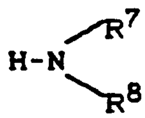

(VI)

in welcher

$R^7$, $R^8$ die oben angegebene Bedeutung haben,

gegebenenfalls nach Aktivierung der Carboxylgruppe über das entsprechende Säurechlorid (beispielsweise durch Thionylchlorid) in üblicher Weise in inerten organischen Lösungsmitteln bei Temperaturen zwischen +10°C und +150°C, bevorzugt zwischen +20°C und +100°C umsetzt.

Die als Ausgangsverbindungen eingesetzten Carbonylverbindungen der allgemeinen Formel (II) sind bekannt oder können nach bekannten Methoden hergestellt werden [GB-Patentschrift 1 235 386 bzw. US-Patentschrift 3 809 691; L. Pentimalli et al., Boll. Sci. Fac. Chim. Ind. Bologna 23, 181 (1965); C.A. 63, 17848 e (1965), D. Bower et al., J. Chem. Soc. 1955, 2834, A. Hetzheim et al., Chem. Ber. 103, 3333 (1970), H. Beyer et al., Z. Chem. 2, 152 (1952) und S. Kano, Yagukagu Zasski 92, 935 (1972)].

Die als Ausgangsverbindungen eingesetzten Phosphonate der Formel (III) sind bekannt oder können nach bekannten

Methoden hergestellt werden [I.Shalek et al. Isr. J. Chem. 7, 585 (1969)].

Die als Ausgangsstoffe eingesetzten Acetamide der Formel (IV) [Verfahren A] sind bekannt und können nach bekannten Methoden hergestellt werden [GB-Patentschrift 715 896 (1954), DBP 1 142 859 (1960)].

Die als Ausgangsstoffe eingesetzten Alkencarbonsäuren der Formel (V) sind neu. Sie können nach bekannten Verfahren hergestellt werden, indem man

[X] Carbonylverbindungen der allgemeinen Formel (II) mit Phosphonaten der allgemeinen Formel (VII)

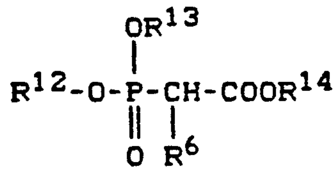

(VII)

in welcher

| | |
|---|---|
| $R^6$, $R^{12}$, $R^{13}$ | die bereits angegebene Bedeutung haben, und |
| $R^{14}$ | für Wasserstoff, Trialkylsilyl, Triarylsilyl oder für Alkyl oder Aralkyl steht, |

in Gegenwart starker Basen in inerten organischen Lösungsmitteln zunächst zu den entsprechenden Alkencarbonsäurederivaten umsetzt und diese dann mit Säuren oder Basen zu den freien Carbonsäuren verseift, wie es von W.S. Wadsworth et al. in J. Am. Chem. Soc. 83, 1733 (1961) beschrieben wird,

oder indem man

[Y] Carbonylverbindungen der allgemeinen Formel (II), in denen $R^1$-$R^4$ die angegebene Bedeutung haben, $R^5$ jedoch für Wasserstoff steht, mit Malonsäuren der allgemeinen Formel (VIII)

$$R^6\text{-}CH(COOH)_2 \qquad (VIII),$$

in welcher

$R^6$ die angegebene Bedeutung hat,

oder mit Meldrumsäure in Gegenwart inerter organischer Lösungsmittel, gegebenenfalls in Gegenwart von Kondensationsmitteln umsetzt, wie es beispielsweise von G. Jones in Organic Reactions 15, 204 ff. beschrieben ist.

Die als Ausgangsverbindungen eingesetzten Phosphonate der Formel (VII) sind bekannt [J. Bontagy, R. Thomas, Chem. Rev. 74, 87 (1974)].

Als starke Basen bei der Durchführung der Verfahren [A] bzw. [X] können die üblichen starken Basen verwendet werden.

Hierzu gehören bevorzugt Alkalihydride wie z.B. Natriumhydrid oder Kaliumhydrid, Alkalialkoholate wie z.B. Natriummethylat, Kaliummethylat, Natriumethylat, Kaliumethylat oder Kalium-tert.-butylat, oder Alkalimetallalkyle wie Methyllithium oder Butyllithium, oder Phenyllithium oder Lithiumdiisopropylamid.

Die bei der Durchführung des Verfahrens [B] eingesetzten sauren oder basischen Katalysatoren sind bevorzugt organische Amine wie beispielsweise Trialkylamine (Triethylamin), Dialkylamine (Diisopropylamin), Pyridin, Piperidin, N-Methyl-piperidin oder auch Gemische der genannten Amine, sowie anorganische Säuren wie Schwefelsäure oder Halogenwasserstoffsäuren, insbesondere Salzsäure, oder Carbonsäureanhydride wie Acetanhydrid.

Die für die Umsetzung mit Aminen (VI) zweckmäßige Aktivierung der freien Carboxylgruppe der Carbonsäuren (V) (Verfahren C) erfolgt vorzugsweise über das entsprechende Carbonsäurechlorid unter Verwendung von Halogenidbildnern wie Thionylchlorid, Phosphortrichlorid oder Phosphorpentachlorid.

Die bei der Durchführung des Verfahrens [Y] verwendeten Kondensationsmittel sind vorzugsweise Pyridin, Pyridinderivate wie Dialkylaminopyridine, Chinolin, Isochinolin, Dialkylamine wie Diisopropylamin oder Dibutylamin, Triethylamin, Pyrrolidin, Piperidin oder N-Methylpiperidin sowie ähnliche stickstoffhaltige organische Basen.

Bei allen Verfahren können als Lösungsmittel die üblichen inerten organischen Lösungsmittel verwendet werden, die sich unter den Versuchsbedingungen nicht verändern.

Hierzu gehören vorzugsweise Ether wie Diethylether, Tetrahydrofuran, Dioxan, Glykolmonomethylether, Glykoldimethylether oder Alkohole wie Methanol, Ethanol, Propanol, Butanol oder Benzylalkohol, Dimethylsulfoxid, Dimethylformamid, Hexamethylphosphorsäuretriamid, oder Kohlenwasserstoffe wie Benzol, Toluol, Xylol oder Erdölfraktionen, oder Basen wie Pyridin, Chinolin oder Picolin. Ebenso ist es möglich, Gemische der genannten Lösungsmittel zu verwenden.

Die erfindungsgemäßen Verbindungen zeichnen sich überraschenderweise durch starke biologische Wirkung aus. Insbesondere besitzen sie ausgeprägte diuretische und saluretische Wirkungen und können daher als Diuretika, Saluretika und Antihypertensiva verwendet werden. Bei Tierversuchen an Ratten zeigt sich, daß die erfindungsgemäßen Verbindungen bei oraler Applikation bereits bei geringen Dosierungen eine ausgeprägte diuretische und saluretische Wirkung bei gleichzeitiger guter Verträglichkeit besitzen. Bei Kenntnis des Standes der Technik konnten diese vorteilhaften Eigenschaften nicht erwartet werden.

Die pharmakologische Wirkung der erfindungsgemäßen Verbindungen wurden nach folgender Testmethode ermittelt: Nüchterne männliche Ratten im Gewicht von 150 bis 250 g (SPF, Wistar, je n=4 Paare) werden mit 10 ml/kg p.o. Tylose-Suspension (0,5 %) als Kontrollen bzw. mit 100 mg/kg p.o. Prüfsubstanz in 10 ml/kg p.o. Tylose-Suspension mittels Schlundsonde behandelt. Die Tiere werden in Stoffwechselkäfige gebracht und die Harn- und Elektrolytauscheidung über 6 Stunden bestimmt ($Na^+$ und $K^+$ Bestimmung: IL-Flammenphotometer).

0225522

Die Ergebnisse einiger erfindungsgemäßer Verbindungen sind in folgender Tabelle beispielhaft aufgeführt:

| Bsp.-Nr. | Harn [ml/kg] | $Na^+$ [µmol/kg] | $K^+$ [µmol/kg] |
|---|---|---|---|
| 14 | 25 | 2900 | 900 |
| 33 | 26 | 3400 | 450 |
| 35 | 21 | 3100 | 800 |

Die neuen erfindungsgemäßen Verbindungen sind als Arzneimittel verwendbare Substanzen. Sie bewirken bei oraler oder parenteraler Anwendung eine Steigerung der Wasser- und Salzausscheidung und können daher zur Behandlung oedematöser und hypertoner Zustände und zur Ausschwemmung toxischer Substanzen dienen.

Darüber hinaus können die Verbindungen bei akutem Nierenversagen eingesetzt werden. Insbesondere zeigen sie auch eine vorteilhafte uricosurische Wirkung.

Die neuen Verbindungen können in bekannter Weise in die üblichen Formulierungen übergeführt werden, wie Tabletten, Kapseln, Dragees, Pillen, Granulate, Sirupe, Emulsionen, Suspensionen und Lösungen, unter Verwendung inerter, nichttoxischer, pharmazeutisch geeigneter Trägersubstanzen oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den unten angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral.

Für den Fall der parenteralen Anwendung können Lösungen der Wirkstoffe unter Verwendung geeigneter flüssiger Trägermaterialien eingesetzt werden. Als besonders vorteilhaft für den Fall der parenteralen Anwendung hat sich die Tatsache herausgestellt, daß die erfindungsgemäßen Verbindungen in Wasser gut lösliche Salze zu bilden vermögen. Derartige Salze können auch für die orale Anwendung der erfindungsgemäßen Verbindungen eine erhöhte Bedeutung besitzen, indem sie die Resorption je nach Wunsch beschleunigen oder verzögern.

Im allgemeinen hat es sich als vorteilhaft erwiesen, bei parenteraler Applikation Mengen von etwa 0,05 bis 100 mg/kg, vorzugsweise etwa 0,1 bis 10 mg/kg Körpergewicht pro Tag zur Erzielung wirksamer Ergebnisse zu verabreichen. Bei oraler Applikation beträgt die Dosierung etwa 0,1 bis 500 mg/kg, vorzugsweise 0,5 bis 100 mg/kg Körpergewicht pro Tag.

Trotzdem kann es gegebenenfalls erforderlich sein ,von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen.

0225522

Herstellungsbeispiele

Beispiel 1

6-(4-Methylpiperazino)-2-phenyl-imidazo[1,2-b]pyridazin

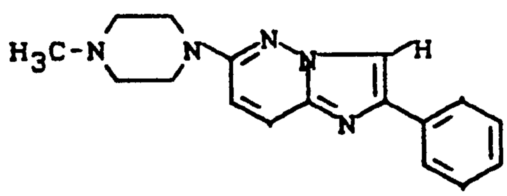

17,2 g 6-Chlor-2-phenyl-imidazo[1,2-b]pyridazin und 40 ml Methylpiperazin werden 12 h im Rückfluß erhitzt. Nach Abkühlen wird die Reaktionsmischung mit Wasser versetzt und 1 h gerührt. Danach wird der Niederschlag abgesaugt, mit Wasser gewaschen und getrocknet.

Schmelzpunkt: 172°C

Ausbeute: 98 % der Theorie.

Analog Beispiel 1 wurden hergestellt:

Beispiel 2

6-Diethylamino-2-phenyl-imidazo[1,2-b]pyridazin

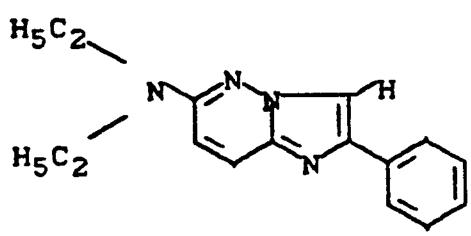

Schmelzpunkt:

Ausbeute: 99 % der Theorie

Beispiel 3

2-Phenyl-6-pyrrolidino-imidazo[1,2-b]pyridazin

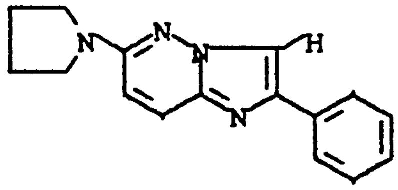

Schmelzpunkt: 192° C
Ausbeute: 98 % der Theorie

Beispiel 4

6-Morpholino-2-phenyl-imidazo[1,2-b]pyridazin

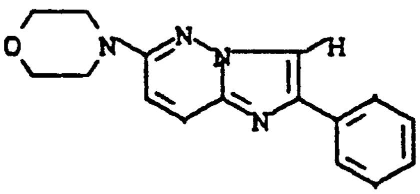

Schmelzpunkt: 181° C
Ausbeute: 84 % der Theorie

Beispiel 5

2-Phenyl-6-piperidino-imidazo[1,2-b]pyridazin

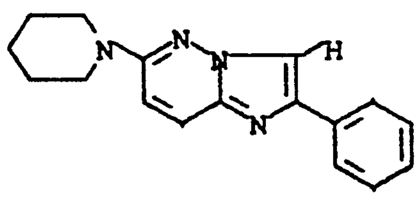

Schmelzpunkt: 164° C
Ausbeute: 89 % der Theorie

Beispiel 6

3-Formyl-6-(4-methylpiperazino)-2-phenyl-imidazo[1,2-b]-pyridazin

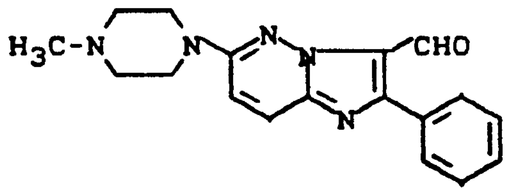

Unter Kühlung werden zu 40 ml DMF 24,5 g $POCl_3$ zugetropft. Dann gibt man weiter 70 ml DMF und 23 g 6-(4-Methylpiperazino)-2-phenyl-imidazo[1,2-b]pyridazin unter Rühren zu, rührt 15 min bei Raumtemperatur und erwärmt anschließend 2 h auf 80° C. Nach Abkühlen wird der Niederschlag abgesaugt und mit DMF gewaschen. Der getrocknete Niederschlag wird in Wasser gelöst, filtriert und das Filtrat mit NaOH alkalisch gestellt. Nach 30 min. Rühren wird das ausgefallene Produkt abgesaugt, mit Wasser gewaschen und getrocknet.

Ausbeute: 50 % der Theorie
Schmelzpunkt: 160° C.

Analog Beispiel 6 wurden hergestellt:

Beispiel 7

3-Formyl-2-phenyl-6-pyrrolidino-imidazo[1,2-b]pyridazin

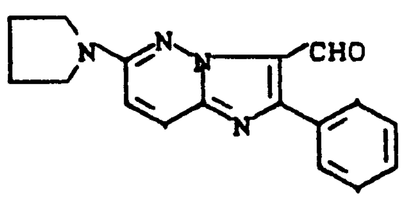

Schmelzpunkt: 192° C
Ausbeute: 63 % der Theorie

Beispiel 8

3-Formyl-6-morpholino-2-phenyl-imidazo[1,2-b]pyridazin

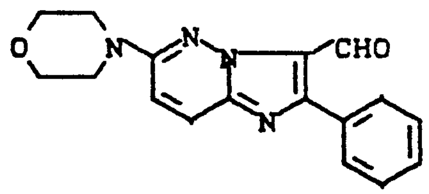

Schmelzpunkt: 209° C
Ausbeute: 44 % der Theorie

Beispiel 9

3-Formyl-2-phenyl-6-piperidino-imidazo[1,2-b]pyridazin

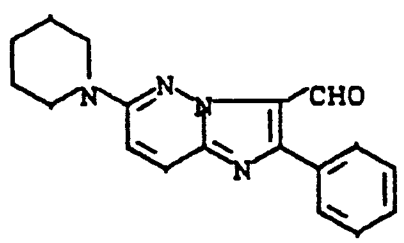

Schmelzpunkt: 161° C
Ausbeute: 62 % der Theorie

Beispiel 10

3-Formyl-6-methyl-2-phenyl-imidazo[1,2-b]pyridazin

0225522

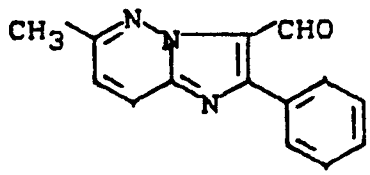

Schmelzpunkt: 166° C
Ausbeute: 76 % der Theorie.

Beispiel 11

2-(6-Methyl-2-phenyl-imidazo[1,2-b]pyridazin-3-yl)propensäure

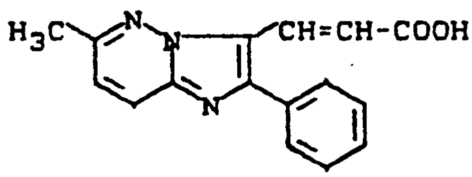

4.74 g 3-Formyl-6-methyl-2-phenyl-imidazo[1,2-b]pyridazin, 3.12 g Malonsäure, 6 ml Pyridin und 1/2 ml Piperidin werden 10 h im Rückfluß erhitzt. Nach Abkühlen wird mit etwas Wasser versetzt, der Niederschlag abgesaugt und mit Wasser gewaschen. Danach wird der Rückstand in Wasser aufgeschlämmt, mit 1 N NaOH alkalisch gestellt. Nach 10 min. Rühren wird vom Ungelösten abfiltriert, das Filtrat mit 1 N HCl angesäuert und der Niederschlag abgesaugt und getrocknet. Ausbeute: 48 % der Theorie.

Schmelzpunkt: 260° C (Zers.)

Beispiel 12

2-(2-Phenyl-6-piperidino-imidazo[1,2-b]pyridazin-3-yl)propensäure-N,N-diethylamid

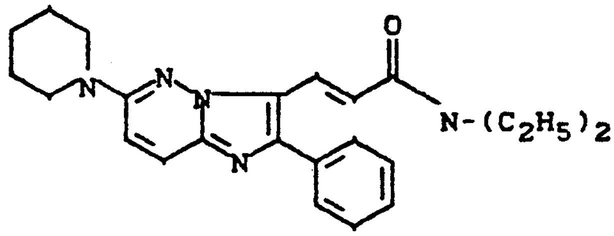

Zu 50 ml Benzol werden 0,4 g Natriumhydrid (80 %ig in Öl) gegeben. Dann tropft man 3 g Diethylphosphonoessigsäurediethylamid zu, wobei die Temperatur bei 20° C gehalten wird. Nachdem der Ansatz 1 h bei 20° C gerührt wurde, gibt man portionsweise 3,1 g 3-Formyl-2-phenyl-6-piperidino-imidazo[1,2-b]pyridazin zu und rührt 1 h bei 45° C. Dann kühlt man ab, dekantiert die Benzollösung vom Niederschlag ab, wäscht die Benzolphase 2 mal mit Wasser und trocknet die organische Phase über $Na_2SO_4$. Danach wird das Lösungsmittel im Vakuum eingedampft und der Rückstand aus Essigester umkristallisiert.
Ausbeute: 69 % der Theorie.
Schmelzpunkt: 199° C.

Beispiel 13

2-(6-Methyl-2-phenyl-imidazo[1,2-b]pyridazin-3-yl)propensäure-N-methyl-N-propylamid

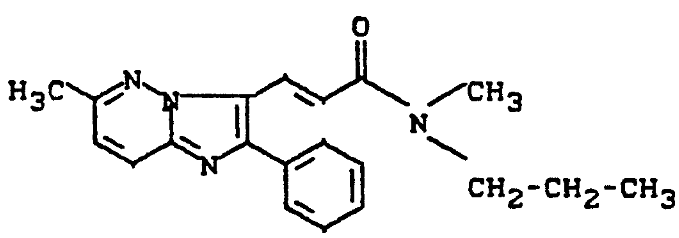

2,8 g 3-Formyl-6-methyl-2-phenyl-imidazo[1,2-b]pyridazin werden in 50 ml Toluol bei 60-70° C suspendiert. Man tropft 2,5 g Thionylchlorid zu, wobei eine klare Lösung entsteht.

Danach erhitzt man 2 h im Rückfluß und kühlt auf Raumtemperatur ab. Nun werden 7.3 g N-Methylpropylamin zugetropft und 1 h unter Rückfluß erhitzt. Der abgekühlte Ansatz wird eingedampft, der Rückstand in $CHCl_3/H_2O$ aufgenommen, die organische Phase abgetrennt und getrocknet. Danach wird das Lösungsmittel im Vakuum eingedampft, der Rückstand aus Essigester umkristallisiert.

Ausbeute: 54 % der Theorie

Schmelzpunkt: 146°C

Analog Beispiel 12 und 13 wurden die in folgender Tabelle aufgeführten Verbindungen hergestellt:

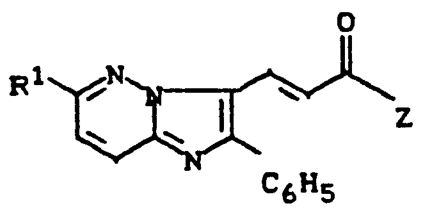

| Bsp.-Nr. | $R^1$ | Z | Schmelzpunkt [°C] |
|---|---|---|---|
| 14 | $CH_3$ | 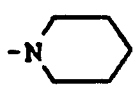 | 147 |
| 15 | $CH_3$ | $-N(C_2H_5)_2$ | 104 |
| 16 | 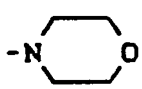 | 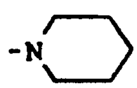 | 184 |
| 17 | $CH_3$ | 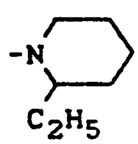 | 106 |

| Bsp.-Nr. | $R^1$ | Z | Schmelzpunkt [°C] |
|---|---|---|---|
| 18 | 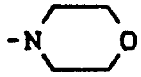 | 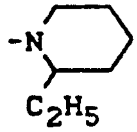 | 173 |
| 19 | $CH_3$ | 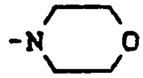 | 183 |
| 20 | 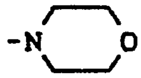 | 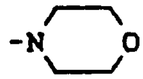 | 120 |
| 21 | 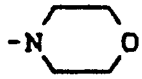 | $-N(C_2H_5)_2$ | 158 |
| 22 | 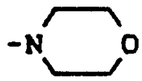 | 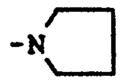 | 215 |
| 23 | 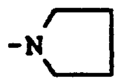 | 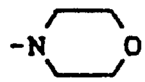 | 241 |
| 24 | 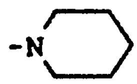 | 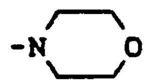 | 178 |
| 25 | 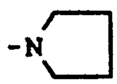 | 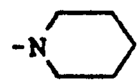 | 258 |
| 26 | 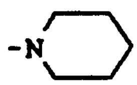 | 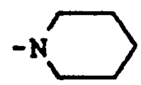 | 178 |
| 27 | 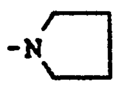 | $-N(C_2H_5)_2$ | 188 |
| 28 | 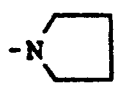 | 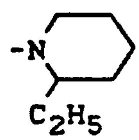 | 142 |
| 29 | 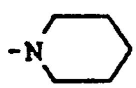 | 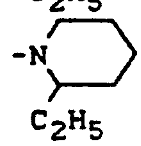 | 128 |
| 30 | $CH_3$ | 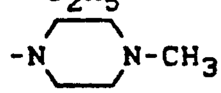 | 136 |

| Bsp.-Nr. | $R^1$ | Z | Schmelzpunkt [°C] |
|---|---|---|---|
| 31 | $CH_3$ | 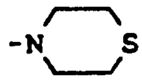 | 164 |
| 32 | 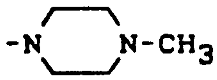 | 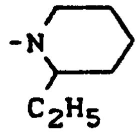 | 144 |
| 33 | 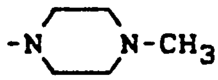 | 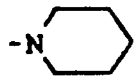 | 206 |
| 34 | 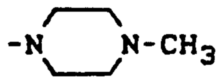 | 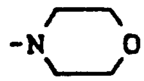 | 214 |
| 35 | 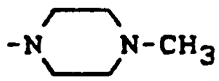 | $-N(C_2H_5)_2$ | 184 |
| 36 | $CH_3$ | 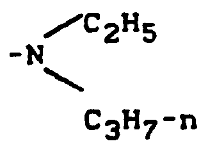 | 127 |
| 37 | $CH_3$ | 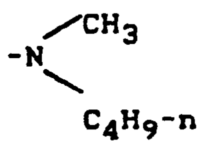 | 141 |
| 38 | $CH_3$ | 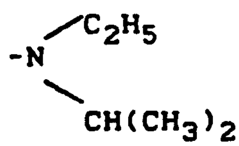 | 134 |
| 39 | 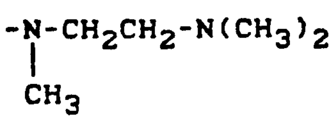 | 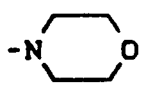 | 124 |
| 40 | 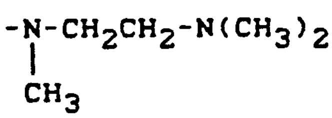 | 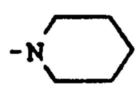 | 144 |
| 41 | 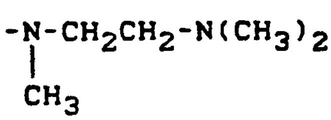 | $-N(C_2H_5)_2$ | 99 |

| Bsp.-Nr. | $R^1$ | Z | Schmelzpunkt [°C] |
|---|---|---|---|
| 42 | $-N-CH_2CH_2-N(CH_3)_2$<br>$CH_3$ | 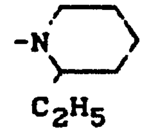 | 116 |
| 43 | $OCH_3$ | 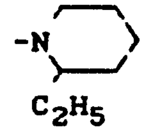 | 140 |
| 44 | 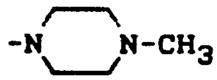 | 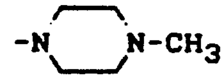 | 161 |
| 45 | 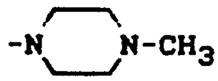 | 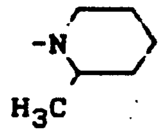 | 211 |
| 46 | 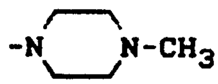 | $-N(CH_2CH_2OH)_2$ | 209 |
| 47 | 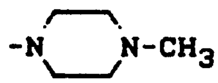 | $-N-C_4H_9(n)$ | 187 |
| 48 | 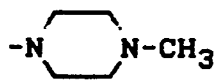 | $-N-CH_2CH_2-N(CH_3)_2$<br>$CH_3$ | 128 |
| 49 | 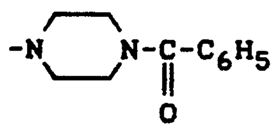 | 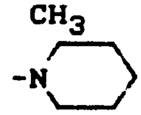 | 234 |
| 50 | 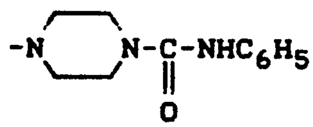 | 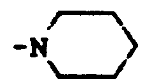 | 198 |
| 51 | 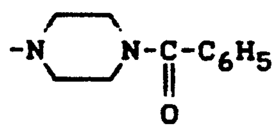 | 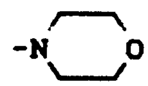 | 188 |
| 52 | 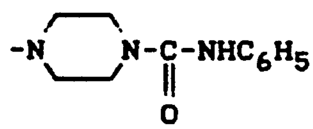 | 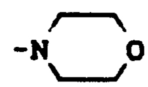 | 208 |
| 53 | 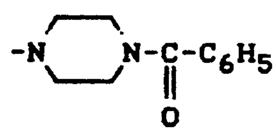 | $-N(C_2H_5)_2$ | 197 |

| Bsp.-Nr. | $R^1$ | Z | Schmelzpunkt [°C] |
|---|---|---|---|
| 54 | 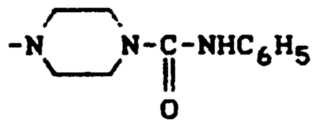 | $-N(C_2H_5)_2$ | 168 |
| 55 | 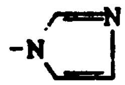 | 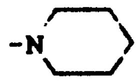 | 234 |
| 56 | 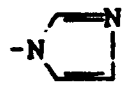 | 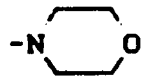 | 228 |

Analog Beispiel 1 wurden hergestellt:

## Beispiel 57

6-Hydrazino-2-phenyl-imidazo[1,2-b]pyridazin

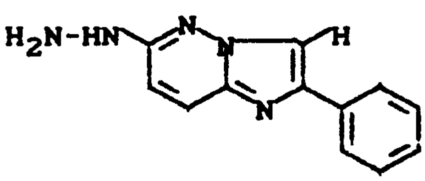

Schmelzpunkt: 198° C

## Beispiel 58

6-Imidazo-2-phenyl-imidazo[1,2-b]pyridazin

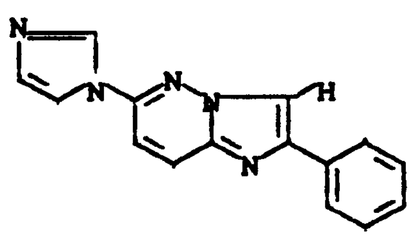

Schmelzpunkt: 202° C

Beispiel 59

6-[4-(2-Hydroxyethyl)piperazino]-2-phenyl-imidazo[1,2-b]pyridazin

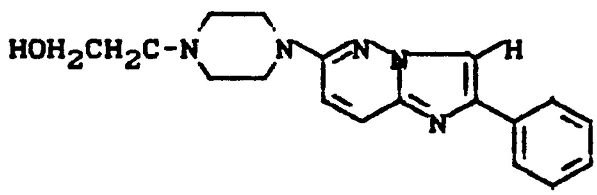

Schmelzpunkt: 154° C

Beispiel 60

6-Piperazino-2-phenyl-imidazo[1,2-b]pyridazin

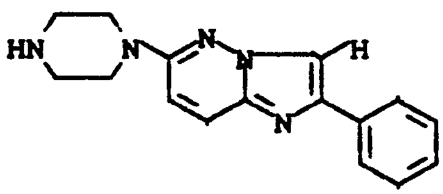

Schmelzpuntk: 124° C

Beispiel 61

6-(4-Benzoylpiperazino)-2-phenyl-imidazo[1,2-b]pyridazin

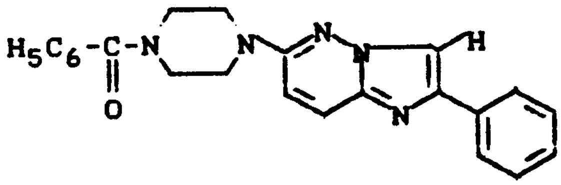

Schmelzpunkt: 224° C

Beispiel 62:

2-Phenyl-6-(4-phenylcarbamoylpiperazino)-imidazo[1,2-b]-pyridazin

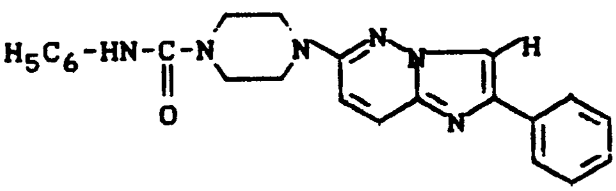

Schmelzpunkt: 226° C

Analog Beispiel 11 wurde hergestellt:

Beispiel 63:

2-[2-Phenyl-6-(4-methylpiperazino)-imidazo[1,2-b]pyridazin

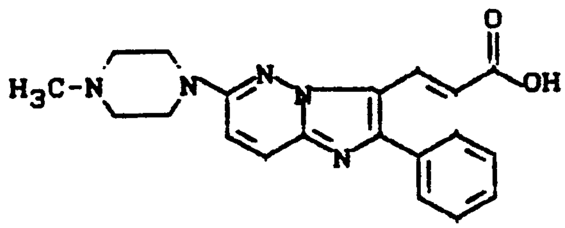

Schmelzpunkt: 244° C

Patentansprüche

1. Imidazopyridazin-alkensäureamide der allgemeinen Formel (I)

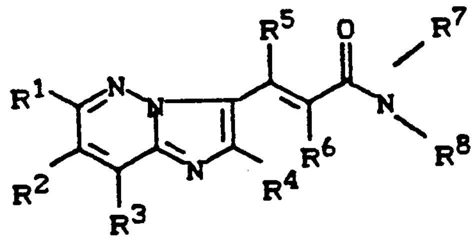

(I)

in welcher

$R^1$ - für Wasserstoff oder
- für Alkoxy oder
- für gegebenenfalls durch Phenyl, Cyano, Hydroxy oder Halogen substituiertes Alkyl oder Alkenyl steht, wobei die Kohlenstoffkette des Alkylrestes gegebenenfalls durch O, S, NH, N-Alkyl, N-Aryl oder N-Aralkyl unterbrochen ist, oder

- für einen Aryl- oder Heterocyclylrest steht, der gegebenenfalls ein, zwei oder drei gleiche oder verschiedene Substituenten aus der Gruppe Alkyl, Alkoxy, Alkoxy, Alkylthio, Trifluormethyl, Trifluormethoxy, Halogen, Nitro, Cyano oder Alkylsulfonyl trägt, oder

- für eine Gruppe $SO_n$-Alkyl steht, worin n 0, 1 oder 2 bedeutet, oder

- für eine Gruppe dэr Formel

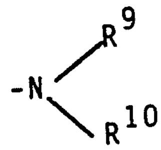 oder 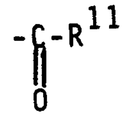 steht,

wobei

$R^9$, $R^{10}$ - gleich oder verschieden sind und
- für Wasserstoff, oder
- für gegebenenfalls durch O, S, NH oder N-Alkyl in der Kette unterbrochenes Alkyl stehen, oder
- für Aryl oder Aralkyl stehen, oder
- für Amino, Alkylamino oder Arylamino stehen,

$R^9$ und $R^{10}$ gemeinsam mit dem Stickstoffatom einen 5- bis 7-gliedrigen gesättigten oder ungesättigten Ring bilden, der gegebenenfalls ein oder zwei Sauerstoff-, Schwefel- und/oder Stickstoffatome als weitere Heteroatome enthalten kann, wobei diese Stickstoffatome gegebenenfalls durch Alkyl, Aryl, Aralkyl, Hydroxyalkyl, Acyl, Alkylaminocarbonyl oder Arylaminocarbonyl substituiert sind,

und wobei

$R^{11}$ - für Wasserstoff,
- für Hydroxy,
- für Alkyl oder Alkenyl,
- für Alkoxy oder Alkenyloxy,
- für Aryloxy oder Aralkoxy steht, oder
- für eine Gruppe der Formel

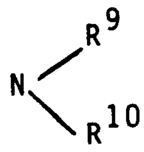 steht,

wobei
$R^9$ und $R^{10}$ die bereits angegebene Bedeutung haben,

$R^2$, $R^3$ - gleich oder verschieden sind und
- für Wasserstoff oder Alkyl stehen,

$R^4$ - für einen Aryl- oder Heterocyclylrest steht, der gegebenenfalls bis zu zweifach gleich oder verschieden durch Alkyl, Halogen, Nitro, Cyano, Alkoxy, Alkylsulfonyl oder Trifluormethyl substituiert ist,

$R^5$ - für Wasserstoff, Alkyl oder Trifluormethyl steht,

$R^6$ - für Wasserstoff, Cyano, Halogen oder
- für Alkoxy, Alkyl oder Sulfonylalkyl, oder
- für die Gruppe $-\underset{\overset{\|}{O}}{C}-R^{11}$ steht, wobei

$R^{11}$ die bereits angegebene Bedeutung hat,

und

$R^7$, $R^8$ - gleich oder verschieden sind und
- für Wasserstoff,
- für Aryl, oder
- für einen geradkettigen, verzweigten oder cyclischen gesättigten oder ungesättigten Kohlenwasserstoffrest mit bis zu 10 C-Atomen

stehen, der gegebenenfalls durch O, S, NH, N-Alkyl, N-Aryl oder N-Aralkyl unterbrochen ist, und der gegebenenfalls substituiert ist durch Hydroxy, Alkoxy, Trifluormethyl, Halogen, Phenyl, Alkoxycarbonyl oder Dialkylamino, wobei die beiden Alkylreste gegebenenfalls gemeinsam mit dem Stickstoffatom einen 5 - 7-gliedrigen Ring bilden, der gegebenenfalls durch O, S, NH oder N-Alkyl unterbrochen ist und wobei die vorgenannten Alkyl- sowie Phenylreste ihrerseits gegebenenfalls durch Halogen, Trifluormethyl, Alkyl, Aryl, Aralkyl, Alkoxy, Alkylthio, Alkylsulfonyl oder Cyano substituiert sind,

oder

$R^7$ und $R^8$ gemeinsam mit dem Stickstoffatom einen 3- bis 8-gliedrigen, gesättigten oder ungesättigten Ring bilden, der gegebenenfalls ein oder zwei O, S, N, NH, N-Alkyl, N-Aryl oder N-Aralkyl enthält, der gegebenenfalls bis zu drei Substituenten aus der Reihe Alkyl, Halogen, Aryl, Aralkyl, Alkoxycarbonyl, Hydroxyalkyl, Alkoxyalkyl oder Trifluormethyl tragen kann, oder der mit einem gegebenenfalls substituierten aromatischen Ring kondensiert sein kann,

sowie ihre physiologisch unbedenklichen Salze, sowie ihre stereoisomeren Formen in Form von Enantiomeren, Diastereomeren und E/Z-Isomeren.

2. Imidazopyridazinalkensäureamide der allgemeinen Formel (I) in Anspruch 1, in welcher

$R^1$ - für Wasserstoff oder

- für $C_1$-$C_8$-Alkoxy steht, oder
- für gegebenenfalls durch Phenyl, Cyano, Hydroxy, Fluor, Chlor oder Brom substituiertes $C_1$-$C_6$-Alkyl, $C_2$-$C_6$-Alkenyl, Cyclopentyl oder Cyclohexyl steht, wobei die Kohlenstoffkette des Alkylrestes gegebenenfalls durch O,S,NH, N-$C_1$-$C_6$-Alkyl, N-Phenyl oder N-Benzyl unterbrochen ist, oder

- für einen Phenyl-, Naphthyl-, Furyl-, Thienyl-, Pyrimidyl-, Pyrazinyl-, Pyridyl-, Chinolyl- oder Isochinolylrest steht, der gegebenenfalls durch ein oder zwei gleiche oder verschiedene Substituenten der Reihe $C_1$-$C_6$-Alkyl, $C_1$-$C_6$-Alkoxy, Fluor, Chlor, Brom, Nitro, Cyano, Trifluormethyl, Trifluormethoxy oder $C_1$-$C_6$-Alkylsulfonyl substituiert ist, oder

- für eine Gruppe $SO_n$-Alkyl steht,

wobei
n für 0 oder 2 steht und Alkyl bis zu 6 C-Atome hat,

oder

- für einen Gruppe der Formel 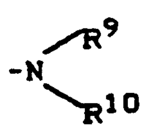 oder 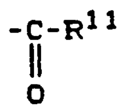 steht,

wobei

$R^9$, $R^{10}$ - gleich oder verschieden sind und
- für Wasserstoff, oder
- für $C_1$-$C_6$-Alkyl stehen, das gegebenenfalls durch O, S, NH oder N-$C_1$-$C_6$-Alkyl unterbrochen ist, oder

- für Phenyl, Naphthyl oder Benzyl stehen, oder
- für Amino, $C_1$-$C_4$-Alkylamino oder Phenylamino steht,

$R^9$ und $R^{10}$ gemeinsam mit dem Stickstoffatom einen 5- bis 7-gliedrigen gesättigten oder ungesättigten Ring bilden, der gegebenenfalls ein oder zwei Sauerstoff-, Schwefel- und/oder Stickstoffatome als weitere Heteroatome enthalten kann, wobei diese Stickstoffatome gegebenenfalls durch $C_1$-$C_6$-Alkyl, $C_1$-$C_6$-Hydroxyalkyl, Benzoyl, Acetyl, $C_1$-$C_4$-Alkylaminocarbonyl oder Phenylaminocarbonyl, Phenyl oder Benzyl substituiert sind,

und wobei

$R^{11}$ - für Wasserstoff,
- für Hydroxy,
- für $C_1$-$C_6$-Alkyl, $C_2$-$C_6$-Alkenyl,
- für $C_1$-$C_6$-Alkoxy, $C_2$-$C_6$-Alkenyloxy,
- für Phenoxy oder Benzyloxy steht, oder
- für eine Gruppe der Formel

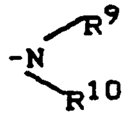 steht, wobei $R^9$ und $R^{10}$ die bereits angegebene Bedeutung haben,

$R^2$, $R^3$ - gleich oder verschieden sind und
- für Wasserstoff oder $C_1$-$C_6$-Alkyl stehen,

$R^4$ - für gegebenenfalls durch $C_1$-$C_6$-Alkyl, Fluor, Chlor, Brom, Nitro, Cyano, $C_1$-$C_6$-Alkoxy, $C_1$-$C_6$-Alkylsulfonyl oder Trifluormethyl substituiertes Phenyl, Naphthyl, Thienyl, Furyl, Pyridyl, Pyrimidyl, Chinolyl oder Isochinolyl steht,

$R^5$ - für Wasserstoff, Trifluormethyl oder $C_1$-$C_6$-Alkyl steht,

$R^6$ - für Wasserstoff, Cyano, Fluor, Chlor, Brom,
- für $C_1$-$C_6$-Alkyl oder
- für die Gruppe $-\underset{\underset{O}{\|}}{C}-R^{11}$ steht, wobei $R^{11}$ die bereits angegebene Bedeutung hat,

und

$R^7$, $R^8$ - gleich oder verschieden sind und
- für Wasserstoff,
- für Phenyl oder

- für einen geradkettigen, verzweigten oder cyclischen Kohlenwasserstoffrest mit bis zu 8 C-Atomen stehen, der gegebenenfalls durch O, S, NH, N-$C_1$-$C_6$-Alkyl, N-Phenyl oder N-Benzyl unterbrochen ist, und der gegebenenfalls substituiert ist durch Hydroxy, $C_1$-$C_6$-Alkoxy, Trifluormethyl, Fluor, Chlor, Brom, Phenyl, $C_1$-$C_6$-Alkoxycarbonyl, Di-$C_1$-$C_6$-Alkylamino, Piperidin, Piperazin, Morpholin, Thiomorpholin, N-Methylpiperazin oder Pyrrolidin, wobei die vorgenannten Alkyl sowie Phenylreste ihrerseits gegebenenfalls durch Fluor, Chlor, Brom, Trifluormethyl, $C_1$-$C_6$-Alkyl, Phenyl, Benzyl, $C_1$-$C_6$-Alkoxy, $C_1$-$C_6$-Alkylsulfonyl oder Cyano substituiert sind,

oder

$R^7$ und $R^8$ gemeinsam mit dem Stickstoffatom einen 5- bis 7-gliedrigen Ring bilden, der gegebenenfalls 1 oder 2 weitere Heteroatome aus der Gruppe Sauerstoff, Schwefel oder Stickstoff enthält, wobei der Stickstoff gegebenenfalls durch Wasserstoff, $C_1$-$C_6$-Alkyl, Phenyl oder Benzyl substituiert ist und wobei der Ring durch bis zu drei gleiche oder verschiedene Substituenten aus der

Reihe $C_1$-$C_6$-Alkyl, Fluor, Chlor, Brom, Phenyl, Benzyl, $C_1$-$C_6$-Alkoxycarbonyl, Hydroxy-$C_1$-$C_6$-alkyl, $C_1$-$C_4$-Alkoxy-$C_1$-$C_6$-alkyl oder Trifluormethyl substituiert sein kann, oder wobei der Ring mit einem gegebenenfalls substituierten aromatischen Ring kondensiert sein kann,

sowie ihre physiologisch unbedenklichen Salze, sowie ihre stereoisomeren Formen in Form von Enantiomeren, Diastereomeren und E/Z-Isomeren.

3. Imidazopyridazinalkensäureamide der allgemeinen Formel (I) in Anspruch 1, in welcher

$R^1$ - für Wasserstoff oder
- für geradkettiges oder verzweigtes $C_1$-$C_4$-Alkoxy oder
- für gegebenenfalls durch Fluor, Chlor oder Hydroxy substituiertes, geradkettiges oder verzweigtes $C_1$-$C_6$-Alkyl steht, wobei die Alkylkette gegebenenfalls durch O,S,NH, N-$C_1$-$C_4$-Alkyl, N-Phenyl oder N-Benzyl unterbrochen ist, oder

- für einen Phenyl-, Naphthyl-, Furyl-, Thienyl-, Pyrimidyl-, oder Pyridylrest steht, oder

- für eine Gruppe der Formel 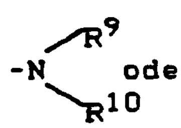 oder 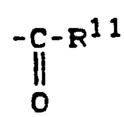

steht,
wobei

$R^9$, $R^{10}$ - gleich oder verschieden sind und
- für Wasserstoff stehen, oder
- für geradkettiges oder verzweigtes $C_1$-$C_6$-Alkyl stehen, das gegebenenfalls durch O, S, NH oder N-$C_1$-$C_3$-Alkyl unterbrochen ist, oder
- für Phenyl, Benzyl oder
- für Amino stehen,

oder

$R^9$ und $R^{10}$ gemeinsam mit dem Stickstoffatom einen 5- bis 6-gliedrigen, gesättigten oder ungesättigten Ring bilden, der gegebenenfalls ein oder zwei Sauerstoff-, Schwefel- und/ oder Stickstoffatome als weitere Heteroatome enthalten kann, wobei diese Stickstoffatome gegebenenfalls durch $C_1$-$C_3$-Alkyl, $C_1$-$C_3$-Hydroxyalkyl, Benzoyl, Acetyl, $C_1$-$C_3$-Alkylaminocarbonyl oder Phenylaminocarbonyl, Phenyl oder Benzyl substituiert sind,

und wobei

$R^{11}$ - für Wasserstoff,
- für Hydroxy,
- für geradkettiges oder verzweigtes $C_1$-$C_4$-Alkyl,
- für geradkettiges oder verzweigtes $C_1$-$C_4$-Alkoxy oder
- für Phenoxy oder Benzyloxy steht, oder

$R^2$, $R^3$ - gleich oder verschieden sind und
- für Wasserstoff oder
- für geradkettiges oder verzweigtes $C_1$-$C_4$-Alkyl stehen,

$R^4$ - für gegebenenfalls durch Fluor, Chlor, Nitro, Cyano, $C_1$-$C_4$-Alkyl, $C_1$-$C_4$-Alkoxy oder Trifluormethyl substituiertes Phenyl, Thienyl oder Pyridyl steht,

$R^5$, $R^6$ - gleich oder verschieden sind und
- für Wasserstoff oder
- für geradkettiges oder verzweigtes $C_1$-$C_4$-Alkyl stehen,

und

$R^7$, $R^8$ - gleich oder verschieden sind und
- für Wasserstoff,
- für Phenyl oder
- für einen geradkettigen, verzweigten oder cyclischen Kohlenwasserstoffrest mit bis zu 6 C-Atomen stehen, der gegebenenfalls durch O, S, NH, N-$C_1$-$C_4$-Alkyl, N-Phenyl oder N-Benzyl unterbrochen ist, und der gegebenenfalls substituiert ist durch Hydroxy, $C_1$-$C_4$-Alkoxy, Trifluormethyl, Fluor, Chlor, Phenyl, Di-$C_1$-$C_4$-alkylamino, Piperidin, Piperazin, N-Methylpiperazin, Morpholin oder Pyrrolidin,

wobei die vorgenannten Alkyl-oder Phenylreste ihrerseits gegebenenfalls durch Fluor, Chlor, Trifluormethyl, $C_1$-$C_4$-Alkyl oder $C_1$-$C_4$-Alkoxy substituiert sind,

oder

$R^7$ und $R^8$ gemeinsam mit dem Stickstoffatom einen 5- bis 7-gliedrigen Ring bilden, der gegebenenfalls 1 oder zwei weitere Heteroatome aus der Gruppe Sauerstoff, Schwefel oder Stickstoff enthält, wobei der Stickstoff gegebenenfalls durch Wasserstoff, $C_1$-$C_4$-Alkyl, Phenyl oder Benzyl substituiert ist und wobei der Ring durch bis zu zwei gleiche oder verschiedene Substituenten aus der Reihe $C_1$-$C_4$-Alkyl, Fluor, Chlor, $C_1$-$C_4$-Hydroxyalkyl, $C_1$-$C_4$-Alkoxy-$C_1$-$C_4$-alkyl oder Trifluormethyl substituiert sein kann oder wobei der Ring mit einem aromatischen Ring kondensiert sein kann,

sowie ihre physiologisch unbedenklichen Salze, sowie ihre stereoisomeren Formen in Form von Enantiomeren, Distereomeren und E/Z-Isomeren.

4. Verbindung der Formel

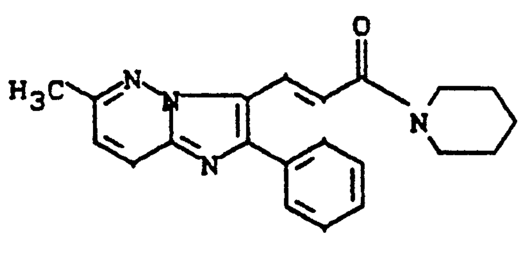

oder

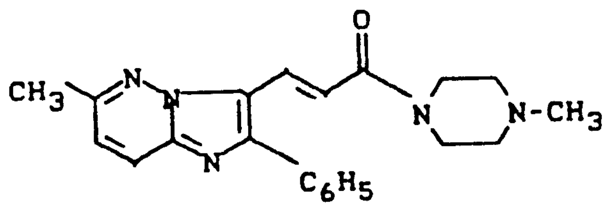

oder

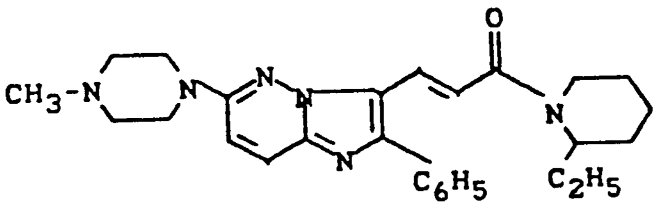

oder

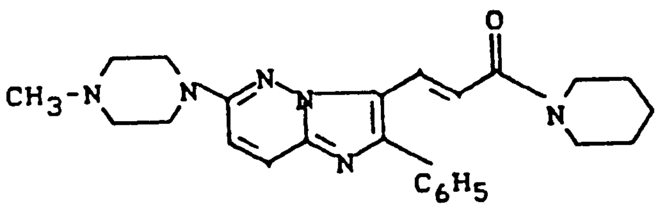

oder

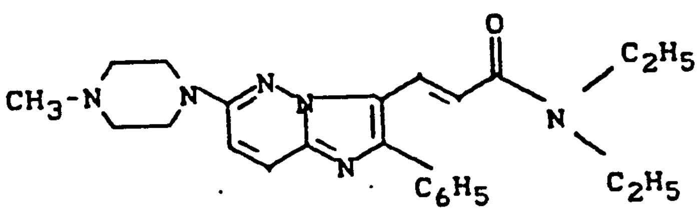

5. Imidazopyridazin-alkensäureamide der allgemeinen Formel (I)

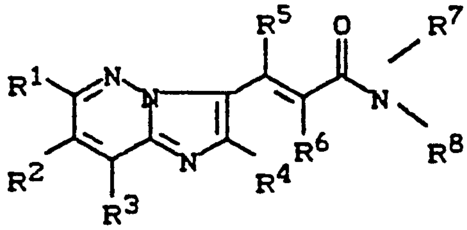

(I)

in welcher

$R^1$ - für Wasserstoff oder
- für Alkoxy oder
- für gegebenenfalls durch Phenyl, Cyano, Hydroxy oder Halogen substituiertes Alkyl oder Alkenyl steht, wobei die Kohlenstoffkette des Alkylrestes gegebenenfalls durch O, S, NH, N-Alkyl, N-Aryl oder N-Aralkyl unterbrochen ist, oder

- für einen Aryl- oder Heterocyclylrest steht, der gegebenenfalls ein, zwei oder drei gleiche oder verschiedene Substituenten aus der Gruppe Alkyl, Alkoxy, Alkylthio, Trifluormethyl, Trifluormethoxy, Halogen, Nitro, Cyano oder Alkylsulfonyl trägt, oder

- für eine Gruppe $SO_n$-Alkyl steht, worin n 0, 1 oder 2 bedeutet,
- oder für eine Gruppe der Formel

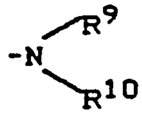 oder 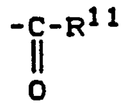 steht,

wobei

$R^9$, $R^{10}$ - gleich oder verschieden sind und
- für Wasserstoff, oder
- für gegebenenfalls durch O, S, NH oder N-Alkyl in der Kette unterbrochenes Alkyl stehen, oder
- für Aryl oder Aralkyl stehen, oder
- für Amino, Alkylamino oder Arylamino stehen,

oder

$R^9$ und $R^{10}$ gemeinsam mit dem Stickstoffatom einen 5- bis 7-gliedrigen, gesättigten oder ungesättigten Ring bilden, der gegebenenfalls ein oder zwei Sauerstoff-, Schwefel- und/oder Stickstoffatome als weitere Heteroatome enthalten kann, wobei dise Stickstoffatome gegebenenfalls durch Alkyl, Aryl, Aralkyl, Hydroxyalkyl, Acyl; Alkylaminocarbonyl substituiert ist,

und wobei

$R^{11}$ - für Wasserstoff,
- für Hydroxy,
- für Alkyl oder Alkenyl,
- für Alkoxy oder Alkenyloxy,
- für Aryloxy oder ARalkoxy steht, oder
- für eine Gruppe der Formel

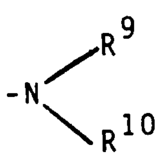 steht, wobei $R^9$ und $R^{10}$ die

bereits angegebene Bedeutung haben,

$R^2$, $R^3$ - gleich oder verschieden sind und
- für Wasserstoff oder Alkyl stehen,

$R^4$ - für einen Aryl- oder Heterocyclylrest steht, der gegebenenfalls bis zweifach gleich oder verschieden durch Alkyl, Halogen, Nitro, Cyano, Alkoxy, Alkylsulfonyl oder Trifluormethyl substituiert ist,

$R^5$ - für Wasserstoff, Alkyl oder Trifluormethyl steht,

$R^6$ - für Wasserstoff, Cyano, Halogen oder
- für Alkoxy, Alkyl oder Sulfonylalkyl, oder
- für die Gruppe $-\underset{\underset{O}{\|}}{C}-R^{11}$ steht, wobei

$R^{11}$ die bereits angegebene Bedeutung hat,

und

$R^7$, $R^8$ - gleich oder verschieden sind und
- für Wasserstoff,
- für Aryl, oder
- für einen geradkettigen, verzweigten oder cyclischen gesättigten oder ungesättigten Kohlenwasserstoffrest mit bis zu 10 C-Atomen

stehen, der gegebenenfalls durch O, S, NH, N-Alkyl, N-Aryl oder N-Aralkyl unterbrochen ist, und der gegebenenfalls substituiert ist durch Hydroxy, Alkoxy, Trifluormethyl, Halogen, Phenyl, Alkoxycarbonyl oder Dialkylamino, wobei die beiden Alkylreste gegebenenfalls gemeinsam mit dem Stickstoffatom einen 5- bis 7-gliedrigen Ring bilden, der gegebenenfalls durch O, S, NH oder N-Alkyl unterbrochen ist und wobei die vorgenannten Alkyl- sowie Phenylreste ihrerseits gegebenenfalls durch Halogen, Trifluormethyl, Alkyl, Aryl, Aralkyl, Alkoxy, Alkylthio, Alkylsulfonyl oder Cyano substituiert sind,

oder

$R^7$ und $R^8$ gemeinsam mit dem Stickstoffatom einen 3- bis 8-gliedrigen, gesättigten oder ungesättigten Ring bilden, der gegebenenfalls ein oder zwei O, S, N, NH, N-Alkyl, N-Aryl oder N-Aralkyl enthält, der gegebenenfalls bis zu drei Substituenten aus der Reihe Alkyl, Halogen, Aryl, Aralkyl, Alkoxycarbonyl, Hydroxyalkyl, Alkoxyalkyl oder Trifluormethyl tragen kann, oder der mit einem gegebenenfalls substituierten aromatischen Ring kondensiert sein kann,

sowie ihre physiologisch unbedenklichen Salze, sowie ihre stereoisomeren Formen in Form von Enantiomeren, Diastereomeren und E/Z-Isomeren, bei der Verwendung zur Behandlung von Krankheiten.

6. Verfahren zur Herstellung von Imidazopyridazinalkensäureamiden der allgemeinen Formel (I)

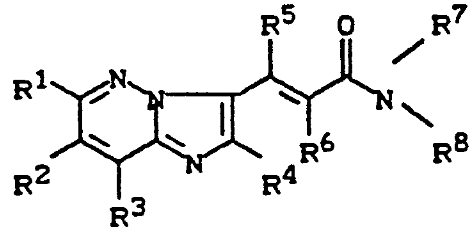

(I)

in welcher

$R^1$ - für Wasserstoff oder
- für Alkoxy oder
- für gegebenenfalls durch Phenyl, Cyano, Hydroxy oder Halogen substituiertes Alkyl oder Alkenyl steht, wobei die Kohlenstoffkette des Alkylrestes gegebenenfalls durch O, S, NH, N-Alkyl, N-Aryl oder N-Aralkyl unterbrochen ist, oder

- für einen Aryl- oder Heterocyclylrest steht, der gegebenenfalls ein, zwei oder drei gleiche oder verschiedene Substituenten aus der Gruppe Alkyl, Alkoxy, Alkylthio, Trifluormethyl, Trifluormethoxy, Halogen, Nitro, Cyano oder Alkylsulfonyl trägt, oder

- für eine Gruppe $SO_n$-Alkyl steht,
worin
n 0, 1 oder 2 bedeutet,

oder

- für eine Gruppe der Formel

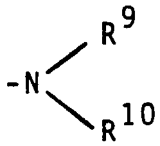 oder 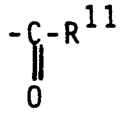 steht,

wobei

$R^9$, $R^{10}$ - gleich oder verschieden sind und
- für Wasserstoff, oder
- für gegebenenfalls durch O, S, NH oder N-Alkyl in der Kette unterbrochenes Alkyl stehen, oder
- für Aryl oder Aralkyl stehen, oder
- für Amino, Alkylamino oder Arylamino stehen,

oder

$R^9$ und $R^{10}$ gemeinsam mit dem Stickstoffatom einen 5- bis 7-gliedrigen, gesättigten oder ungesättigten Ring bilden, der gegebenenfalls ein oder zwei Sauerstoff-, Schwefel- und/oder Stickstoffatome als weitere Heteroatome enthalten kann, wobei diese Stickstoffatome gegebenenfalls durch Alkyl, Aryl, Aralkyl, Hydroxyalkyl, Acyl, Alkylaminocarbonyl oder Arylaminocarbonyl substituiert ist,

und wobei

$R^{11}$ - für Wasserstoff,
- für Hydroxy,
- für Alkyl oder Alkenyl,
- für Alkoxy oder Alkenyloxy,
- für Aryloxy oder aralkyloxy steht, oder
- für eine Gruppe der Formel

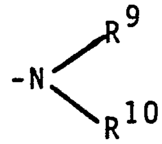 steht, wobei $R^9$ und $R^{10}$ die bereits angegebene Bedeutung haben,

$R^2$, $R^3$ - gleich oder verschieden sind und
- für Wasserstoff oder Alkyl stehen,

$R^4$ - für einen Aryl- oder Heterocyclylrest steht, der gegebenenfalls bis zu zweifach gleich oder verschieden durch Alkyl, Halogen, Nitro, Cyano, Alkoxy, Alkylsulfonyl oder Trifluormethyl substituiert ist,

$R^5$ - für Wasserstoff, Alkyl oder Trifluormethyl steht,

$R^6$ - für Wasserstoff, Cyano, Halogen oder
- für Alkoxy, Alkyl oder Sulfonylalkyl, oder
- für die Gruppe $-\underset{\underset{O}{\|}}{C}-R^{11}$ steht, wobei

$R^{11}$ die bereits angegebene Bedeutung hat,

und

$R^7$, $R^8$ - gleich oder verschieden sind und
- für Wasserstoff,
- für Aryl, oder
- für einen geradkettigen, verzweigten oder cyclischen gesättigten oder ungesättigten Kohlenwasserstoffrest mit bis zu 10 C-Atomen

stehen, der gegebenenfalls durch O, S, NH, N-Alkyl, N-Aryl oder N-Aralkyl unterbrochen ist, und der gegebenenfalls substituiert ist durch Hydroxy, Alkoxy, Trifluormethyl, Halogen, Phenyl, Alkoxycarbonyl oder Dialkylamino, wobei die beiden Alkylreste gegebenenfalls gemeinsam mit dem Stickstoffatom einen 5 - 7-gliedrigen Ring bilden, der gegebenenfalls durch O, S, NH oder N-Alkyl unterbrochen ist und wobei die vorgenannten Alkyl- sowie Phenylreste ihrerseits gegebenenfalls durch Halogen, Trifluormethyl, Alkyl, Aryl, Aralkyl, Alkoxy, Alkylthio, Alkylsulfonyl oder Cyano substituiert sind,

oder

$R^7$ und $R^8$ gemeinsam mit dem Stickstoffatom einen 3- bis 8-gliedrigen, gesättigten oder ungesättigten Ring bilden, der gegebenenfalls ein oder zwei O, S, N, NH, N-Alkyl, N-Aryl oder N-Aralkyl enthält, der gegebenenfalls bis zu drei Substituenten aus der Reihe Alkyl, Halogen, Aryl, Aralkyl, Alkoxycarbonyl, Hydroxyalkyl, Alkoxyalkyl oder Trifluormethyl tragen kann, oder der mit einem gegebenenfalls substituierten aromatischen Ring kondensiert sein kann,

sowie ihren physiologisch unbedenklichen Salzen, sowie ihre stereoisomeren Formen in Form von Enantiomeren, Diastereomeren und E/Z-Isomeren, dadurch gekennzeichnet, daß man entweder

[A] Carbonylverbindungen der allgemeinen Formel (II),

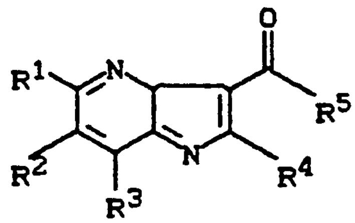

(II)

in welcher

$R^1$ - $R^5$ die oben angegebene Bedeutung haben,

mit Phosphonaten der allgemeinen Formel (III)

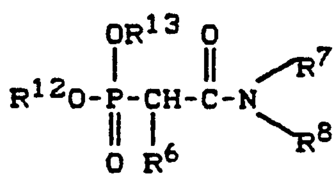

(III)

in welcher

$R^6$ - $R^8$ die oben angegebene Bedeutung haben und

$R^{12}$, $R^{13}$ für gegebenenfalls substituiertes Alkyl oder Aralkyl stehen,

in Gegenwart starker Basen in inerten organischen Lösungsmitteln bei Temperaturen zwischen +20° C und +200° C, bevorzugt zwischen +20° C und +150° C umsetzt,

oder daß man

[B] Carbonylverbindungen der allgemeinen Formel (II) mit Acetamiden der allgemeinen Formel (IV),

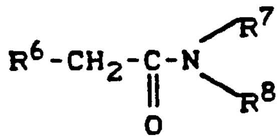

in welcher

$R^6$ - $R^8$ die oben angegebene Bedeutung haben,

in Gegenwart von sauren oder basischen Katalysatoren und gegebenenfalls in Anwesenheit inerter organischer Lösungsmittel bei Temperaturen zwischen 20° C und 200° C, bevorzugt zwischen 20° C und 150° C umsetzt,

oder daß man

[C] Alkencarbonsäuren der allgemeinen Formel (V)

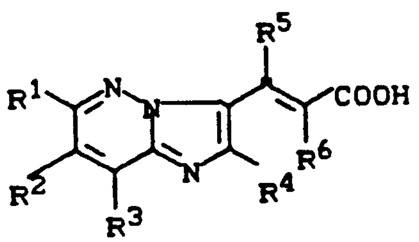

(V)

in welcher

$R^1$ -$R^6$ die oben angegebene Bedeutung haben,

mit Aminen der allgemeinen Formel (VI)

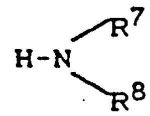 (VI)

in welcher

$R^7$, $R^8$ die oben angegebene Bedeugtung haben,

gegebenenfalls nach Aktivierung der Carboxylgruppe über das entsprechende Säurechlorid in inerten organischen Lösungsmitteln bei Temperaturen zwischen +10° C und +150° C, bevorzugt zwischen +20° C und +100° C amidiert.

7. Alkencarbonsäuren der allgemeinen Formel (V)

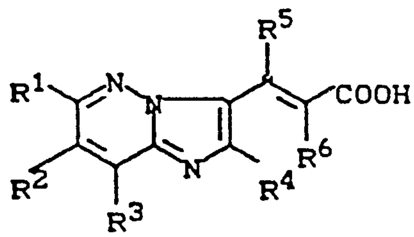 (V)

in welcher

$R^1$-$R^6$ die in Anspruch 6 angegebene Bedeutung haben.

8. Verfahren zur Herstellung von Alkencarbonsäuren der allgemeinen Formel (V)

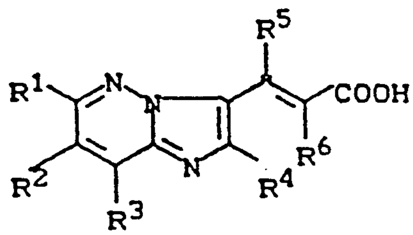 (V)

in welcher

$R^1$-$R^6$ die in Anspruch 6 angegebene Bedeutung haben,

dadurch gekennzeichnet, daß man entweder Carbonylverbindungen der allgemeinen Formel (II) in Anspruch 10 mit Phosphonaten der allgemeinen Formel (VII)

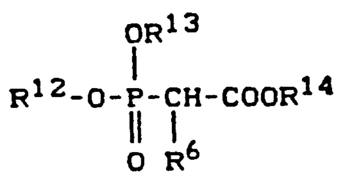

(VII)

in welcher

$R^6$, $R^{12}$, $R^{13}$ die in Anspruch 6 angegebene Bedeutung haben, und

$R^{14}$ für Wasserstoff, Trialkylsilyl, Triarylsilyl oder für Alkyl oder Aralkyl steht,

in Gegenwart starker Basen in inerten organischen Lösungsmitteln zunächst zu den entsprechenden Alkencarbonsäurederivaten umsetzt und diese dann mit Säuren oder Basen zu den freien Carbonsäure verseift,

oder indem man Carbonylverbindungen der allgemeinen Formel (II), in denen $R^1$-$R^4$ die in Anspruch 10 angegebene Bedeutung haben, $R^5$ jedoch für Wasserstoff steht, mit Malonsäuren der allgemeinen Formel (VIII)

$$R^6\text{-}CH(COOH)_2 \qquad (VIII)$$

in welcher

$R^6$ die angegebene Bedeutung hat,

oder mit Meldrumsäure in Gegenwart inerter organischer Lösungsmittel, gegebenenfalls in Gegenwart von Kondensationsmitteln umsetzt.

9. Arzneimittel, gekennzeichnet durch einen Gehalt an mindestens einem Imidazopyridazinalkensäureamid der allgemeinen Formel (I) in Anspruch 1 oder deren physiologisch unbedenklichen Salzen.

10. Verwendung von Imidazopyridazin-alkensäureamiden der allgemeinen Formel (I) in Anspruch 1 oder deren physiologisch unbedenklichen Salze zur Behandlung von Krankheiten.

11. Verwendung von Imidazopyridazin-alkensäureamiden der allgemeinen Formel (I) in Anspruch 1 oder deren physiologisch unbedenklichen Salze als Antihypertensiva, Diuretika und Saluretika.

12. Verwendung von Imidazopyridazin-alkensäureamiden der allgemeinen Formel (I) in Anspruch 1 oder deren physiologisch unbedenklichen Salze zur Herstellung von Antihypertensiva, Diuretika und Saluretika.

Europäisches Patentamt

**EUROPÄISCHER RECHERCHENBERICHT**

0225522

Nummer der Anmeldung

EP 86 11 6123

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP-A-0 041 215 (BAYER)<br>* Ansprüche 1,7; Seite 18, Zeilen 1-13 * & US-A-4 444 770<br>----- | 1,9 | C 07 D 487/04<br>A 61 K 31/50 //<br>(C 07 D 487/04<br>C 07 D 237:00<br>C 07 D 235:00 ) |

| RECHERCHIERTE SACHGEBIETE (Int. Cl.4) |
|---|
| C 07 D 487/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 11-03-1987 | ALFARO I. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82